(19) Europäisches Patentamt
European Patent Office
Office européen des brevets

(11) Veröffentlichungsnummer: **0 093 951**
**B1**

(12) **EUROPÄISCHE PATENTSCHRIFT**

(45) Veröffentlichungstag der Patentschrift: **29.10.86**

(21) Anmeldenummer: **83104103.3**

(22) Anmeldetag: **27.04.83**

(51) Int. Cl.⁴: **C 07 D 487/04**, A 61 K 31/495, A 61 K 31/50 // (C07D487/04, 241:00, 235:00), (C07D487/04, 237:00, 235:00)

(54) **2-Aryl-Tetraazaindene.**

(30) Priorität: **11.05.82 DE 3217583**

(43) Veröffentlichungstag der Anmeldung: **16.11.83 Patentblatt 83/46**

(45) Bekanntmachung des Hinweises auf die Patenterteilung: **29.10.86 Patentblatt 86/44**

(84) Benannte Vertragsstaaten: **AT BE CH DE FR GB IT LI NL SE**

(56) Entgegenhaltungen:
**EP - A - 0 022 495**
**DE - A - 2 118 319**
**US - A - 3 244 715**

**Die Akte enthält technische Angaben, die nach dem Eingang der Anmeldung eingereicht wurden und die nicht in dieser Patentschrift enthalten sind.**

(73) Patentinhaber: **Merck Patent Gesellschaft mit beschränkter Haftung, Frankfurter Strasse 250, D-6100 Darmstadt (DE)**

(72) Erfinder: **Jonas, Rochus, Dr., Telemannweg 24, D-6100 Darmstadt (DE)**
Erfinder: **Kloft, Michael, Dr., Kollwitzweg 27, D-6100 Darmstadt-Arheilgen (DE)**
Erfinder: **Wurziger, Hanns, Dr., Greinstrasse 7b, D-6100 Darmstadt-Arheilgen (DE)**
Erfinder: **Harting, Jürgen, Dr., Rodinghweg 15, D-6100 Darmstadt (DE)**
Erfinder: **Enenkel, Hans Joachim, Dr., Wingertsbergstrasse 5, D-6100 Darmstadt (DE)**
Erfinder: **Minck, Klaus-Otto, Dr., Büchestrasse 8, D-6105 Ober-Ramstadt (DE)**
Erfinder: **Schliep, Hans-Jochen, Dr., Weingartenstrasse 16, D-6109 Traisa (DE)**

EP 0 093 951 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 2-Aryl-tetraazaindene der allgemeinen Formel I

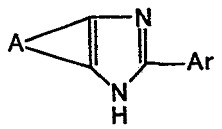

I

worin

–A– –N=CH–CH=N– oder –CH=N–N=CH–,

Ar einen ein- bis dreifach durch Hydroxy-, Mercapto-, Dialkylamino-, Trifluormethyl- und/oder –Z–R-Gruppen substituierten Phenylrest,

Z –O–, –S– oder –SO– und

R Alkyl, Alkenyl, Alkinyl oder Cyanmethyl bedeuten,

wobei die Alkyl-, Alkenyl- und Alkinylgruppen jeweils bis zu 5 C-Atome besitzen

sowie ihre physiologisch unbedenklichen Salze.

Diese Verbindungen umschliessen die 1,3,4,7-Tetraazaindene (Imidazo(4,5-b)pyrazine; vgl. «The Ring Index», 2nd Edition, American Chemical Society, 1960, Nr. 1176; Formel Ia) und die 1,3,5,6-Tetraazaindene (Imidazo(4,5-d)pyridazine; vgl. «The Ring Index», 1.c., Nr. 1177; Formel Ib):

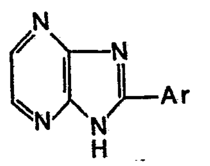

Ia

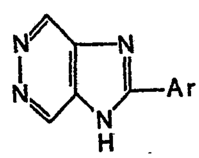

Ib

Eine Verbindung, die der Formel Ib entspricht, worin Ar die Phenylgruppe bedeutet, ist aus J. Het. Chem. Band 1, Seiten 182–185 (1964) bekannt. Über eine pharmakologische Wirkung dieser Verbindung wird dort nichts ausgesagt.

Ähnliche Verbindungen sind aus der DE-OS 2 305 339 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I und ihrer physiologisch unbedenklichen Salze gelöst.

Es wurde gefunden, dass diese Verbindungen bei guter Verträglichkeit wertvolle pharmakologische Eigenschaften besitzen. Insbesondere zeigen sie eine Wirkung auf den Blutdruck, die Herzkraft (positif inotrope Wirksamkeit) und eine Ulcus-Wirkung. Die Blutdruck- und Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen oder Katze ermittelt werden, z.B. nach Methoden, wie sie in Arzneimittelforschung, Band 31 (I) Nr. 1a (1981), Seiten 141 bis 170, beschrieben sind.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind Verbindungen der Formel I und ihre physiologisch unbedenklichen Salze.

In den Verbindungen der Formel I kann die Phenylgruppe einfach (in o-, m- oder p-Stellung), zweifach (in 2,3-, 2,4-, 2,5-, 2,6-, 3,4- oder 3,5-Stellung) oder dreifach (in 2,3,4-, 2,3,5-, 2,3,6- oder 3,4,5-Stellung) substituiert sein. Bevorzugt sind als Rest Ar 2,4-disubstituierte, ferner o- oder p-monosubstituierte Phenylgruppen.

Alkyl ist vorzugsweise unverzweigt, hat vorzugsweise 1–4, insbesondere 1–3 C-Atome und steht bevorzugt für Methyl, ferner für Ethyl, Propyl, Isopropyl, Butyl, Isobutyl, sek.-Butyl, tert.-Butyl, Pentyl oder Isopentyl.

Alkenyl ist vorzugsweise unverzweigt, hat insbesondere 2–4, bevorzugt 3 C-Atome und steht bevorzugt für Allyl, ferner für Vinyl, 1-Propen-1-yl, Butenyl wie 1-Buten-1-yl, 2-Buten-1-yl, 3-Buten-1-yl, Pentenyl wie 1-Penten-1-yl, 2-Penten-1-yl, 3-Penten-1-yl. Die Alkenylgruppen können aber auch verzweigt sein; Beispiele dafür sind

1-Methyl-2-propen-1-yl,
1-Methyl-2-buten-1-yl,
2-Methyl-3-buten-1-yl,
1,1-Dimethyl-2-propen-1-yl.

Bevorzugte Alkinylgruppen sind unverzweigt und besitzen insbesondere 3, aber auch 2, 4 oder 5 C-Atome wie Propargyl (= 2-Propin-1-yl), ferner Ethinyl, 1-Propin-1-yl, Butinyl wie 1-Butin-1-yl, 2-Butin-1-yl, 3-Butin-1-yl, Pentinyl wie 1-Pentin-1-yl, 2-Pentyn-1-yl, 3-Pentin-1-yl. Die Alkinylgruppen können aber auch verzweigt sein; Beispiele dafür sind

1-Methyl-2-propin-1-yl,
1-Methyl-2-butin-1-yl,
2-Methyl-3-butin-1-yl,
1,1-Dimethyl-2-propin-1-yl.

In den Verbindungen der Formel I sind z.B. folgende Substituenten am Phenylrest besonders bevorzugt: Methoxy, Propargyloxy, Cyanmethoxy; ferner Hydroxy, Mercapto, Dimethylamino, Diethylamino, Trifluormethyl, Ethoxy, Propoxy, Isopropoxy, Butoxy, Vinyloxy, Allyloxy, Methylthio, Ethylthio, Propylthio, Isopropylthio, Butylthio, Vinylthio, Allylthio, Propargylthio, Cyanmethylthio, Methylsulfinyl, Ethylsulfinyl, Propylsulfinyl, Isopropylsulfinyl, Butylsulfinyl, Vinylsulfinyl, Allylsulfinyl, Propargylsulfinyl, Cyanmethylsulfinyl.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ic und Id ausgedrückt werden, die der Formel I entsprechen und worin der Rest Ar einen Phenylrest bedeutet, der

in Ic:

durch 1–3 Alkoxygruppen und/oder eine Trifluormethyl-, Alkenyloxy-, Alkinyloxy-, Alkylthio-oder Cyanmethoxygruppe substituiert ist, wobei die Alkyl-, Alkenyl- und Alkinylgruppen jeweils bis zu 3 C-Atome besitzen;

in Id:
durch zwei Methoxygruppen oder durch eine Methoxygruppe und eine Propargyloxy- oder Cyanmethoxygruppe substituiert ist.

Besonders bevorzugt sind diejenigen Verbindungen der Formel I bzw. Ia bis Id, worin die Substituenten in 2- und/oder 4-Stellung stehen.

Gegenstand der Erfindung ist ferner ein Verfahren zur Herstellung der Verbindungen der Formel I sowie ihrer physiologisch unbedenklichen Salze, dadurch gekennzeichnet, dass man ein Diamin der allgemeinen Formel II

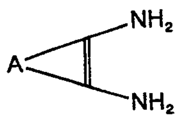

II

worin die Gruppe
–A– die oben angegebene Bedeutung hat
mit einer Benzoesäure der allgemeinen Formel III

HOOC–Ar III

worin
Ar die oben angegebene Bedeutung hat
oder mit einem ihrer reaktionsfähigen Derivate oder mit einem Aldehyd der allgemeinen Formel IV

OCH–Ar IV

worin
Ar die oben angegebene Bedeutung hat
in Gegenwart eines Oxydationsmittels
umsetzt
oder dass man eine Verbindung, die der Formel I entspricht, aber anstelle einer oder mehrerer freier Hydroxy- und/oder Mercaptogruppen eine oder mehrere geschützte Hydroxy- und/oder Mercaptogruppen enthält, mit einem solvolysierenden oder hydrogenolysierenden Mittel behandelt
und dass man gegebenenfalls in dem erhaltenen Produkt Hydroxygruppen verethert und/oder Mercaptogruppen in Thioethergruppen überführt und/oder Thioethergruppen zu Sulfinylgruppen oxydiert und/oder eine erhaltene Verbindung durch Behandeln mit einer Säure in eines ihrer physiologisch unbedenklichen Salze umwandelt.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart; insbesondere aber in der DE-OS 2 305 339) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Vorzugsweise werden die Verbindungen der Formel I erhalten durch Reaktion von II mit Benzoesäuren der Formel III oder ihren reaktionsfähigen Derivaten. Als reaktionsfähige Derivate eignen sich insbesondere die entsprechenden Nitrile, Säurehalogenide, Ester, Amide, Imidsäureester, Imidsäurethioester, Imidsäurehalogenide, Amidine, Thiocarbonsäureester, Dithiocarbonsäureester oder Orthoester.

Die Ausgangsstoffe II und III sind teilweise bekannt. Sofern sie nicht bekannt sind, können sie nach an sich bekannten Methoden hergestellt werden. So sind die Benzoesäuren der Formel III, die –Z–R-Gruppen tragen, beispielsweise durch Veretherung entsprechender Hydroxybenzoesäuren bzw. durch Thioveretherung entsprechender Mercaptobenzoesäuren bzw. durch Oxidation entsprechender thioether-gruppenhaltiger Benzoesäuren erhältlich; die Veretherung bzw. Thioveretherung kann auch stufenweise durchgeführt werden.

Im einzelnen erfolgt die Umsetzung von II mit III in Gegenwart oder Abwesenheit eines inerten Lösungsmittels bei Temperaturen zwischen etwa −20 und etwa 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin, Pyridin oder Picoline; Glykole und Glykolether wie Ethylenglykol, Diethylenglykol, 2-Methoxy-ethanol; Ketone wie Aceton; Ether wie Tetrahydrofuran oder Dioxan; Amide wie Dimethylformamid; Sulfoxide wie Dimethylsulfoxid. Auch Gemische dieser Lösungsmittel sind geeignet. In manchen Fällen empfiehlt sich der Zusatz von katalytischen Mengen einer Säure wie p-Toluolsulfonsäure oder der Zusatz eines Dehydratisierungsmittels wie Phosphoroxychlorid, Polyphosphorsäure oder Thionylchlorid, wobei das Dehydratisierungsmittel auch als Lösungsmittel dienen kann.

Verwendet man die freien Benzoesäuren der Formel III, so wird die Reaktion zweckmässig in Gegenwart eines der genannten Dehydratisierungsmittel und gegebenenfalls einer tertiären Base wie Pyridin oder Triethylamin durchgeführt, vorzugsweise bei Temperaturen zwischen −20 und 150°.

Die Umsetzung kann auch stufenweise durchgeführt werden. So ist es z.B. möglich, II mit einem Säurechlorid der Formel Ar–COCl partiell zum 2-ArCONH-3-amino-pyrazin bzw. 3-ArCONH-4-aminopyridazin zu acylieren, das anschliessend, z.B. mit $POCl_3$, zu I dehydratisiert wird.

Es ist auch möglich, an Stelle von III einen entsprechenden Aldehyd der Formel IV zu verwenden, wenn man gleichzeitig in Gegenwart eines Oxydationsmittels arbeitet. Bevorzugt verwendet man als Oxydationsmittel Schwefel in einem Kohlenwasserstoff wie Benzol, Toluol, Xylol oder Mesitylen oder Natriumdisulfit in Lösungsmitteln wie Dimethylacetamid, jeweils bei Temperaturen zwischen etwa 80 und etwa 200°. Die Aldehyde der Formel IV sind in der Regel neu und beispielsweise durch Veretherung der entsprechenden Hydroxyaldehyde erhältlich.

Verbindungen der Formel I, die freie Hydroxy- und/oder Mercaptogruppen enthalten, sind weiterhin durch Solvolyse oder Hydrogenolyse von entsprechenden Verbindungen erhältlich, in denen die Hydroxy- und/oder Mercaptogruppen durch Schutzgruppen blockiert sind, welche auf diese Weise abgespalten werden können.

Die Ausgangsstoffe für diese Solvolyse bzw. Hydrogenolyse entsprechen der allgemeinen Formel V

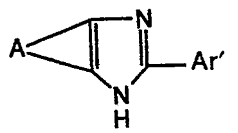

V

worin

Ar′ einen Phenylrest bedeutet, der durch eine bis drei geschützte Hydroxy- und/oder Mercaptogruppen substituiert ist und zusätzlich eine oder zwei freie Hydroxy-, Mercapto-, Dialkylamino-, Trifluormethyl- und/oder Z–R-Gruppen tragen kann, insgesamt jedoch höchstens dreifach substituiert ist, und

A, Z und R die oben angegebene Bedeutung haben.

Die Ausgangsstoffe der Formel V sind beispielsweise erhältlich durch Reaktion von II mit einer Benzoesäure der Formel HOOC–Ar′ oder ihren reaktionsfähigen Derivaten nach den oben angegebenen Methoden.

Als Schutzgruppen kommen die bekannten Hydroxyschutzgruppen und Mercaptoschutzgruppen in Frage. Diese Ausdrücke beziehen sich auf Gruppen, die geeignet sind, Hydroxy- bzw. Mercaptogruppen vor chemischen Umsetzungen zu schützen, die aber leicht entfernbar sind, nachdem die gewünschte chemische Reaktion an einer anderen Stelle des Moleküls durchgeführt worden ist. Typische Schutzgruppen sind beispielsweise leicht spaltbare Ester-, Thioester-, Ether- und Thioethergruppen mit vorzugsweise 1–12 C-Atomen, im einzelnen z.B. Alkanoyl mit vorzugsweise 1–6 C-Atomen wie Acetyl, Aroyl mit vorzugsweise 7–11 C-Atomen wie Benzoyl, unsubstituiertes und substituiertes Aryl und Aralkyl mit jeweils bis zu 11 C-Atomen wie 2,4-Dinitrophenyl, Benzyl, Triphenylmethyl, ferner z.B. Tetrahydropyranyl. Natur und Grösse der Schutzgruppen sind nicht kritisch, da sie nach dem vorliegenden Verfahren abgespalten werden.

Eine Solvolyse der solvolytisch abspaltbaren Schutzgruppen gelingt vorzugsweise in Form der Hydrolyse in wässerigem oder wässerig-alkoholischem Medium in Gegenwart von Säuren wie Salzsäure oder von Basen wie Natrium- oder Kaliumhydroxid bei Temperaturen zwischen etwa 0 und 100°.

Eine Hydrogenolyse der hydrogenolytisch abspaltbaren Schutzgruppen gelingt z.B. in Gegenwart eines Schwermetallkatalysators wie Platin, Palladium oder Nickel in einem inerten Lösungsmittel wie Methanol, Ethanol, Tetrahydrofuran oder Ethylacetat bei Temperaturen zwischen etwa 0 und 100° und Drucken zwischen etwa 1 und 200 bar.

Gewünschtenfalls kann man eine oder mehrere in dem erhaltenen Produkt vorhandene Hydroxygruppe(n) verethern und/oder Mercaptogruppe(n) in Thioethergruppen überführen.

Als Veretherungsmittel bzw. Thioveretherungsmittel eignen sich dafür vorzugsweise solche der allgemeinen Formel VI

X–R VI

worin

X Cl, Br, J, OH, Alkylsulfonyloxy oder Arylsulfonyloxy und

R Alkyl, Alkenyl, Alkinyl oder Cyanmethyl bedeuten,

wobei die Alkyl-, Alkenyl- und Alkinylgruppen jeweils bis zu 5 und die Arylgruppe 6–10 C-Atome besitzen.

Typische Veretherungs- bzw. Thioveretherungsmittel sind z.B. Methyl-, Ethyl-, Propyl-, Isopropyl-, Allyl- oder Propargylchlorid oder -bromid, Chlor- oder Bromacetonitril.

Zur Veretherung bzw. Thioveretherung wird die Hydroxyverbindung bzw. Mercaptoverbindung zweckmässig zunächst in eines ihrer Salze, z.B. das Na-Salz, umgewandelt, das anschliessend mit VI in einem der angegebenen Lösungsmittel bei Temperaturen zwischen etwa 0 und 120° umgesetzt wird. Man kann auch eine freie Hydroxyverbindung mit dem entsprechenden Alkohol VI (X = OH) in Gegenwart von Diethyl-azodicarboxylat/Triphenylphosphin verethern, zweckmässig in einem Lösungsmittel wie Tetrahydrofuran oder Dioxan bei Temperaturen zwischen etwa 10 und etwa 40°.

Ferner kann man, falls erwünscht, eine in einem erhaltenen Produkt der Formel I gegebenenfalls vorhandene Thioethergruppe zu einer Sulfinylgruppe oxydieren, zweckmässig mit Wasserstoffperoxid, Persäuren oder Cr(VI)-Verbindungen wie Chromsäure in Gegenwart eines inerten Lösungsmittels wie Wasser, einem Alkohol (z.B. Methanol oder Ethanol), einer Säure (z.B. Essigsäure) oder einem Keton (z.B. Aceton) bei Temperaturen zwischen etwa −20 und 100°.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks--und/oder Aromastoffe enthalten. Sie können, falls erwünscht, auch einen oder mehrere weitere Wirkstoffe enthalten, z. B. ein oder mehrere Vitamine.

Die Verbindungen der Formel I können bei der Bekämpfung von Krankheiten, insbesondere von Herzinsuffizienz, verwendet werden.

Dabei werden die erfindungsgemässen Substanzen in der Regel in Analogie zu bekannten positiv inotrop wirksamen Substanzen, wie Sulmazol oder Amrinon, verabreicht, vorzugsweise in Dosierungen zwischen etwa 10 und 500 mg, insbesondere zwischen 20 und 100 mg pro Dosierungseinheit. Die tägliche Dosierung liegt vorzugsweise zwischen etwa 0,2 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet «übliche Aufarbeitung»:

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

Beispiel 1

Ein Gemisch von 11 g 2,3-Diaminopyrazin (IIa), 20,6 g 2-Methoxy-4-propargyloxy-benzoesäure [F. 142°; erhältlich durch Reaktion von 2,4-Dihydroxybenzoesäuremethylester mit Propargylchlorid zu 2-Hydroxy-4-propargyloxy-benzoesäuremethylester (F. 100°), Reaktion mit Methyljodid zu 2-Methoxy-4-propargyloxy-benzoesäuremethylester (F. 88°) und Verseifung] und 300 ml $POCl_3$ wird 2 Stunden gekocht. Man dampft ein, versetzt den Rückstand mit Eiswasser und macht mit $K_2CO_3$-Lösung alkalisch. Das ausgefallene 2-(2-Methoxy-4-propargyloxyphenyl)-1,3,4,7-tetraazainden wird abfiltriert. F. 189–190°.

Analog erhält man aus IIa bzw. aus 4,5-Diaminopyridazin (IIb) und den entsprechenden Benzoesäuren der Formel III
(z. B. o-Propargyloxybenzoesäure, F. 82°;
m- Propargyloxybenzoesäure, F. 130°;
p-Propargyloxybenzoesäure, F. 224°;
2-Methoxy-5-propargyloxy-benzoesäure, F. 103°;
3-Methoxy-4-propargyloxy-benzoesäure, F. 195°;
3,5-Dimethoxy-4-propargyloxy-benzoesäure, F. 208°;
2,4-Bis-propargyloxy-benzoesäure, F. 152°),
wobei man die Reaktionstemperatur zwischen 70 und 110° halten kann:

2-o-Hydroxyphenyl-1,3,4,7-tetraazainden
2-m-Hydroxyphenyl-1,3,4,7-tetraazainden
2-p-Hydroxyphenyl-1,3,4,7-tetraazainden
2-o-Mercaptophenyl-1,3,4,7-tetraazainden
2-m-Mercaptophenyl-1,3,4,7-tetraazainden
2-p-Mercaptophenyl-1,3,4,7-tetraazainden
2-o-Dimethylaminophenyl-1,3,4,7-tetraazainden
2-m-Dimethylaminophenyl-1,3,4,7-tetraazainden
2-p-Dimethylaminophenyl-1,3,4,7-tetraazainden
2-o-Diethylaminophenyl-1,3,4,7-tetraazainden
2-m-Diethylaminophenyl-1,3,4,7-tetraazainden
2-p-Diethylaminophenyl-1,3,4,7-tetraazainden
2-o-Trifluormethylphenyl-1,3,4,7-tetraazainden
2-m-Trifluormethylphenyl-1,3,4,7-tetraazainden
2-p-Trifluormethylphenyl-1,3,4,7-tetraazainden, F. 338°
2-o-Methoxyphenyl-1,3,4,7-tetraazainden, F. 165°

2-m-Methoxyphenyl-1,3,4,7-tetraazainden
2-p-Methoxyphenyl-1,3,4,7-tetraazainden, F. 289°
2-o-Ethoxyphenyl-1,3,4,7-tetraazainden
2-m-Ethoxyphenyl-1,3,4,7-tetraazainden
2-p-Ethoxyphenyl-1,3,4,7-tetraazainden
2-o-Propoxyphenyl-1,3,4,7-tetraazainden
2-m-Propoxyphenyl-1,3,4,7-tetraazainden
2-p-Propoxyphenyl-1,3,4,7-tetraazainden
2-p-Isopropoxyphenyl-1,3,4,7-tetraazainden
2-p-Butoxyphenyl-1,3,4,7-tetraazainden
2-p-Isobutoxyphenyl-1,3,4,7-tetraazainden
2-p-Pentoxyphenyl-1,3,4,7-tetraazainden
2-o-Allyloxyphenyl-1-3,4,7-tetraazainden
2-m-Allyloxyphenyl-1,3,4,7-tetraazainden
2-p-Allyloxyphenyl-1,3,4,7-tetraazainden
2-p-Ethinyloxyphenyl-1,3,4,7-tetraazainden
2-o-Propargyloxyphenyl-1,3,4-7-tetraazainden
2-m-Propargyloxyphenyl-1,3,4,7-tetraazainden
2-p-Propargyloxyphenyl-1,3,4,7-tetraazainden, Fumarat, F. 273°
2-(p-3-Pentin-1-yloxy-phenyl)-1,3,4,7-tetraazainden
2-o-Cyanmethoxyphenyl-1,3,4,7-tetraazainden
2-m-Cyanmethoxyphenyl-1,3,4,7-tetraazainden
2-p-Cyanmethoxyphenyl-1,3,4,7-tetraazainden
2-o-Methylthiophenyl-1,3,4,7-tetraazainden
2-m-Methylthiophenyl-1,3,4,7-tetraazainden
2-p-Methylthiophenyl-1,3,4,7-tetraazainden
2-o-Ethylthiophenyl-1,3,4,7-tetraazainden
2-m-Ethylthiophenyl-1,3,4,7-tetraazainden
2-p-Ethylthiophenyl-1,3,4,7-tetraazainden
2-o-Propylthiophenyl-1,3,4,7-tetraazainden
2-m-Propylthiophenyl-1,3,4,7-tetraazainden
2-p-Propylthiophenyl-1,3,4,7-tetraazainden
2-p-Isopropylthiophenyl-1,3,4,7-tetraazainden
2-p-Butylthiophenyl-1,3,4,7-tetraazainden
2-p-Isobutylthiophenyl-1,3,4,7-tetraazainden
2-p-Pentylthiophenyl-1,3,4,7-tetraazainden
2-o-Allylthiophenyl-1,3,4,7-tetraazainden
2-m-Allylthiophenyl-1,3,4,7-tetraazainden
2-p-Allylthiophenyl-1,3,4,7-tetraazainden
2-p-Ethinylthiophenyl-1,3,4,7-tetraazainden
2-o-Propargylthiophenyl-1,3,4,7-tetraazainden
2-m-Propargylthiophenyl-1,3,4,7-tetraazainden
2-p-Propargylthiophenyl-1,3,4,7-tetraazainden
2-o-Cyanmethylthiophenyl-1,3,4,7-tetraazainden
2-m-Cyanmethylthiophenyl-1,3,4,7-tetraazainden
2-p-Cyanmethylthiophenyl-1,3,4,7-tetraazainden
2-o-Methylsulfinylphenyl-1,3,4,7-tetraazainden
2-m-Methylsulfinylphenyl-1,3,4,7-tetraazainden
2-p-Methylsulfinylphenyl-1,3,4,7-tetraazainden
2-o-Ethylsulfinylphenyl-1,3,4,7-tetraazainden
2-m-Ethylsulfinylphenyl-1,3,4,7-tetraazainden
2-p-Ethylsulfinylphenyl-1,3,4,7-tetraazainden
2-o-Propylsulfinylphenyl-1,3,4,7-tetraazainden
2-m-Propylsulfinylphenyl-1,3,4,7-tetraazainden
2-p-Propylsulfinylphenyl-1,3,4,7-tetraazainden
2-p-Isopropylsulfinylphenyl-1,3,4,7-tetraazainden
2-p-Butylsulfinylphenyl-1,3,4,7-tetraazainden
2-p-Isobutylsulfinylphenyl-1-3,4,7-tetraazainden
2-p-Pentylsulfinylphenyl-1,3,4,7-tetraazainden
2-o-Allylsulfinylphenyl-1,3,4,7-tetraazainden
2-m-Allylsulfinylphenyl-1,3,4,7-tetraazainden
2-p-Allylsulfinylphenyl-1,3,4,7-tetraazainden
2-p-Ethinylsulfinylphenyl-1,3,4,7-tetraazainden
2-o-Propargylsulfinylphenyl-1,3,4,7-tetraazainden
2-m-Propargylsulfinylphenyl-1,3,4,7-tetraazainden
2-p-Propargylsulfinylphenyl-1,3,4,7-tetraazainden
2-o-Cyanmethylsulfinylphenyl-1,3,4,7-tetraazainden
2-m-Cyanmethylsulfinylphenyl-1,3,4,7-tetraazainden
2-p-Cyanmethylsulfinylphenyl-1,3,4,7-tetraazainden
2-(2,4-Dihydroxyphenyl)-1,3,4,7-tetraazainden
2-(2-Hydroxy-4-methoxyphenyl)-1,3,4,7-tetraazainden
2-(4-Hydroxy-2-methoxyphenyl)-1,3,4,7-tetraazainden
3-(2-Ethoxy-4-hydroxyphenyl)-1,3,4,7-tetraazainden
2-(4-Ethoxy-2-hydroxyphenyl)-1,3,4,7-tetraazainden
2-(2-Dimethylamino-4-methoxyphenyl)-1,3,4,7-tetraazainden
2-(4-Dimethylamino-2-methoxyphenyl)-1,3,4,7-tetraazainden, F. 279–280°
2-(2-Methoxy-4-trifluormethylphenyl)-1,3,4,7-tetraazainden
2-(4-Methoxy-2-trifluormethylphenyl)-1,3,4,7-tetraazainden
2-(2,3-Dimethoxyphenyl)-1,3,4,7-tetraazainden
2-(2,4-Dimethoxyphenyl)-1,3,4,7-tetraazainden, F. 210–215°; Fumarat, F. 265° (Zers.)
2-(2,5-Dimethoxyphenyl)-1,3,4,7-tetraazainden
2-(2,6-Dimethoxyphenyl)-1,3,4,7-tetraazainden
2-(3,4-Dimethoxyphenyl)-1,3,4,7-tetraazainden, F. 248–249°
2-(3,5-Dimethoxyphenyl)-1,3,4,7-tetraazainden
2-(2-Ethoxy-4-methoxyphenyl)-1,3,4,7-tetraazainden
2-(4-Ethoxy-2-methoxyphenyl)-1,3,4,7-tetraazainden
2-(2,4-Diethoxyphenyl)-1,3,4,7-tetraazainden
2-(2-Isopropyloxy-4-methoxyphenyl)-1,3,4,7-tetraazainden
2-(4-Isopropyloxy-2-methoxyphenyl)-1,3,4,7-tetraazainden
2-(2,4-Diisopropyloxyphenyl)-1,3,4,7-tetraazainden
2-(2-Allyloxy-4-methoxyphenyl)-1,3,4,7-tetraazainden
2-(4-Allyloxy-2-methoxyphenyl)-1,3,4,7-tetraazainden
2-(2,4-Diallyloxyphenyl)-1,3,4,7-tetraazainden
2-(2-Hydroxy-4-propargyloxyphenyl)-1,3,4,7-tetraazainden
2-(4-Hydroxy-2-propargyloxyphenyl)-1,3,4,7-tetraazainden
2-(2-Methoxy-3-propargyloxyphenyl)-1,3,4,7-tetraazainden
2-(2-Methoxy-5-propargyloxyphenyl)-1,3,4,7-tetraazainden
2-(2-Methoxy-6-propargyloxyphenyl)-1,3,4,7-tetraazainden

2-(3-Methoxy-4-propargyloxyphenyl)-1,3,4,7-tetraazainden
2-(4-Methoxy-2-propargyloxyphenyl)-1,3,4,7-tetraazainden
2-(2-Ethoxy-4-propargyloxyphenyl)-1,3,4,7-tetraazainden
2-(4-Ethoxy-2-propargyloxyphenyl)-1,3,5,7-tetraazainden
2-(2-Allyloxy-4-propargyloxyphenyl)-1,3,4,7-tetraazainden
2-(4-Allyloxy-2-propargyloxyphenyl)-1,3,4,7-tetraazainden
2-(2,4-Dipropargyloxyphenyl)-1,3,4,7-tetraazainden
2-(2-Cyanmethoxy-4-hydroxyphenyl)-1,3,4,7-tetraazainden
2-(4-Cyanmethoxy-2-hydroxyphenyl)-1,3,4,7-tetraazainden
2-(2-Cyanmethoxy-4-methoxyphenyl)-1,3,4,7-tetraazainden
2-(4-Cyanmethoxy-2-methoxyphenyl)-1,3,4,7-tetraazainden, F. 232–233°
2-(2-Cyanmethoxy-4-ethoxyphenyl)-1,3,4,7-tetraazainden
2-(4-Cyanmethoxy-2-ethoxyphenyl)-1,3,4,7-tetraazainden
2-(2-Allyloxy-4-cyanmethoxyphenyl)-1,3,4,7-tetraazainden
2-(4-Allyloxy-2-cyanmethoxyphenyl)-1,3,4,7-tetraazainden
2-(2-Cyanmethoxy-4-propargyloxyphenyl)-1,3,4,7-tetraazainden
2-(4-Cyanmethoxy-2-propargyloxyphenyl)-1,3,4,7-tetraazainden
2-(2,4-Bis-cyanmethoxy-phenyl)-1,3,4,7-tetraazainden
2-(2-Mercapto-4-methoxyphenyl)-1,3,4,7-tetraazainden
2-(4-Mercapto-2-methoxyphenyl)-1,3,4,7-tetraazainden
2-(2-Hydroxy-4-methylthiophenyl)-1,3,4,7-tetraazainden
2-(4-Hydroxy-2-methylthiophenyl)-1,3,4,7-tetraazainden
2-(2-Methoxy-4-methylthiophenyl)-1,3,4,7-tetraazainden, F. 198°
2-(2-Methoxy-5-methylthiophenyl)-1,3,4,7-tetraazainden
2-(4-Methoxy-2-methylthiophenyl)-1,3,4,7-tetraazainden
2-(2-Ethoxy-4-methylthiophenyl)-1,3,4,7-tetraazainden
2-(4-Ethoxy-2-methylthiophenyl)-1,3,4,7-tetraazainden
2-(2-Allyloxy-4-methylthiophenyl)-1,3,4,7-tetraazainden
2-(4-Allyloxy-2-methylthiophenyl)-1,3,4,7-tetraazainden
2-(2-Methylthio-4-propargyloxyphenyl)-1,3,4,7-tetraazainden
2-(4-Methylthio-2-propargyloxyphenyl)-1,3,4,7-tetraazainden
2-(2-Cyanmethoxy-4-methylthiophenyl)-1,3,4,7-tetraazainden
2-(4-Cyanmethoxy-2-methylthiophenyl)-1,3,4,7-tetraazainden
2-(2,4-Bis-methylthio-phenyl)-1,3,4,7-tetraazainden
2-(4-Ethylthio-2-methoxyphenyl)-1,3,4,7-tetraazainden
2-(4-Allylthio-2-methoxyphenyl)-1,3,4,7-tetraazainden
2-(4-Methoxy-2-propargylthiophenyl)-1,3,4,7-tetraazainden
2-(4-Cyanmethylthio-2-methoxyphenyl)-1,3,4,7-tetraazainden, F. 204–205°
2-(2-Hydroxy-4-methylsulfinylphenyl)-1,3,4,7-tetraazainden
2-(4-Hydroxy-2-methylsulfinylphenyl)-1,3,4,7-tetraazainden
2-(2-Methoxy-4-methylsulfinylphenyl)-1,3,4,7-tetraazainden
2-(4-Methoxy-2-methylsulfinylphenyl)-1,3,4,7-tetraazainden
2-(2-Ethoxy-4-methylsulfinylphenyl)-1,3,4,7-tetraazainden
2-(4-Ethoxy-2-methylsulfinylphenyl)-1,3,4,7-tetraazainden
2-(2-Allyloxy-4-methylsulfinylphenyl)-1,3,4,7-tetraazainden
2-(4-Allyloxy-2-methylsulfinylphenyl)-1,3,4,7-tetraazainden
2-(2-Methylsulfinyl-4-propargyloxyphenyl)-1,3,4,7-tetraazainden
2-(4-Methylsulfinyl-2-propargyloxyphenyl)-1,3,4,7-tetraazainden
2-(2-Cyanmethoxy-4-methylsulfinylphenyl)-1,3,4,7-tetraazainden
2-(4-Cyanmethoxy-2-methylsulfinylphenyl)-1,3,4,7-tetraazainden
2-(2,4-Bis-methylsulfinyl-phenyl)-1,3,4,7-tetraazainden
2-(4-Ethylsulfinyl-2-methoxyphenyl)-1,3,4,7-tetraazainden
2-(4-Allylsulfinyl-2-methoxyphenyl)-1,3,4,7-tetraazainden
2-(2-Methoxy-4-propargylsulfinyl)-1,3,4,7-tetraazainden
2-(4-Cyanmethylsulfinyl-2-methoxy)-1,3,4,7-tetraazainden
2-(2,3,4,-Trimethoxyphenyl)-1,3,4,7-tetraazainden, F. 216°
2-(3,4,5-Trimethoxyphenyl)-1,3,4,7-tetraazainden
2-(3,5-Dimethoxy-4-propargyloxyphenyl)-1,3,4,7-tetraazainden
2-o-Hydroxyphenyl-1,3,5,6-tetraazainden
2-m-Hydroxyphenyl-1,3,5,6-tetraazainden
2-p-Hydroxyphenyl-1,3,5,6-tetraazainden
2-o-Mercaptophenyl-1,3,5,6-tetraazainden
2-m-Mercaptophenyl-1,3,5,6-tetraazainden
2-p-Mercaptophenyl-1,3,5,6-tetraazainden
2-o-Dimethylaminophenyl-1,3,5,6-tetraazainden
2-m-Dimethylaminophenyl-1,3,5,6-tetraazainden
2-p-Dimethylaminophenyl-1,3,5,6-tetraazainden
2-o-Diethylaminophenyl-1,3,5,6-tetraazainden
2-m-Diethylaminophenyl-1,3,5,6-tetraazainden
2-p-Diethylaminophenyl-1,3,5,6-tetraazainden
2-o-Trifluormethylphenyl-1,3,5,6-tetraazainden
2-m-Trifluormethylphenyl-1,3,5,6-tetraazainden

2-p-Trifluormethylphenyl-1,3,5,6-tetraazainden
2-o-Methoxyphenyl-1,3,5,6-tetraazainden, F. 208°
2-m-Methoxyphenyl-1,3,5,6-tetraazainden
2-p-Methoxyphenyl-1,3,5,6-tetraazainden
2-o-Ethoxyphenyl-1,3,5,6-tetraazainden
2-m-Ethoxyphenyl-1,3,5,6-tetraazainden
2-p-Ethoxyphenyl-1,3,5,6-tetraazainden
2-o-Propoxyphenyl-1,3,5,6-tetraazainden
2-m-Propoxyphenyl-1,3,5,6-tetraazainden
2-p-Propoxyphenyl-1,3,5,6-tetraazainden
2-p-Isopropoxyphenyl-1,3,5,6-tetraazainden
2-p-Butoxyphenyl-1,3,5,6-tetraazainden
2-p-Isobutoxyphenyl-1,3,5,6-tetraazainden
2-p-Pentoxyphenyl-1,3,5,6-tetraazainden
2-o-Allyloxyphenyl-1,3,5,6-tetraazainden
2-m-Allyloxyphenyl-1,3,5,6-tetraazainden
2-p-Allyloxyphenyl-1,3,5,6-tetraazainden
2-p-Ethinyloxyphenyl-1,3,5,6-tetraazainden
2-o-Propargyloxyphenyl-1,3,5,6-tetraazainden
2-m-Propargyloxyphenyl-1,3,5,6-tetraazainden
2-p-Propargyloxyphenyl-1,3,5,6-tetraazainden
2-(p-3-Pentin-1-yloxy-phenyl)-1,3,5,6-tetraazainden
2-o-Cyanmethoxyphenyl-1,3,5,6-tetraazainden
2-m-Cyanmethoxyphenyl-1,3,5,6-tetraazainden
2-p-Cyanmethoxyphenyl-1,3,5,6-tetraazainden
2-o-Methylthiophenyl-1,3,5,6-tetraazainden
2-m-Methylthiophenyl-1,3,5,6-tetraazainden
2-p-Methylthiophenyl-1,3,5,6-tetraazainden
2-o-Ethylthiophenyl-1,3,5,6-tetraazainden
2-m-Ethylthiophenyl-1,3,5,6-tetraazainden
2-p-Ethylthiophenyl-1,3,5,6-tetraazainden
2-o-Propylthiophenyl-1,3,5,6-tetraazainden
2-m-Propylthiophenyl-1,3,5,6-tetraazainden
2-p-Propylthiophenyl-1,3,5,6-tetraazainden
2-p-Isopropylthiophenyl-1,3,5,6-tetraazainden
2-p-Butylthiophenyl-1,3,5,6-tetraazainden
2-p-Isobutylthiophenyl-1,3,5,6-tetraazainden
2-p-Pentylthiophenyl-1,3,5,6-tetraazainden
2-o-Allylthiophenyl-1,3,5,6-tetraazainden
2-m-Allylthiophenyl-1,3,5,6-tetraazainden
2-p-Allylthiophenyl-1,3,5,6-tetraazainden
2-p-Ethinylthiophenyl-1,3,5,6-tetraazainden
2-o-Propargylthiophenyl-1,3,5,6-tetraazainden
2-m-Propargylthiophenyl-1,3,5,6-tetraazainden
2-p-Propargylthiophenyl-1,3,5,6-tetraazainden
2-o-Cyanmethylthiophenyl-1,3,5,6-tetraazainden
2-m-Cyanmethylthiophenyl-1,3,5,6,-tetraazainden
2-p-Cyanmethylthiophenyl-1,3,5,6-tetraazainden
2-o-Methylsulfinylphenyl-1,3,5,6-tetraazainden
2-m-Methylsulfinylphenyl-1,3,5,6-tetraazainden
2-p-Methylsulfinylphenyl-1,3,5,6-tetraazainden
2-o-Ethylsulfinylphenyl-1,3,5,6-tetraazainden
2-m-Ethylsuflinylphenyl-1,3,5,6-tetraazainden
2-p-Ethylsulfinylphenyl-1,3,5,6-tetraazainden
2-o-Propylsulfinylphenyl-1,3,5,6-tetraazainden
2-m-Propylsulfinylphenyl-1,3,5,6-tetraazainden
2-p-Propylsulfinylphenyl-1,3,5,6-tetraazainden
2-p-Isopropylsulfinylphenyl-1,3,5,6-tetraazainden
2-p-Butylsulfinylphenyl-1,3,5,6-tetraazainden
2-p-Isobutylsulfinylphenyl-1,3,5,6-tetraazainden
2-p-Pentylsulfinylphenyl-1,3,5,6-tetraazainden
2-o-Allylsulfinylphenyl-1,3,5,6-tetraazainden
2-m-Allylsulfinylphenyl-1,3,5,6-tetraazainden
2-p-Allylsulfinylphenyl-1,3,5,6-tetraazainden
2-p-Ethinylsulfinylphenyl-1,3,5,6-tetraazainden
2-o-Propargylsulfinylphenyl-1,3,5,6-tetraazainden
2-m-Propargylsulfinylphenyl-1,3,5,6-tetraazainden
2-p-Propargylsulfinylphenyl-1,3,5,6-tetraazainden
2-o-Cyanmethylsulfinylphenyl-1,3,5,6-tetraazainden
2-m-Cyanmethylsulfinylphenyl-1,3,5,6-tetraazainden
2-p-Cyanmethylsulfinylphenyl-1,3,5,6-tetraazainden
2-(2,4-Dihydroxyphenyl)-1,3,5,6-tetraazainden
2-(2-Hydroxy-4-methoxyphenyl)-1,3,5,6-tetraazainden
2-(4-Hydroxy-2-methoxyphenyl)-1,3,5,6-tetraazainden
3-(2-Ethoxy-4-hydroxyphenyl)-1,3,5,6-tetraazainden
2-(4-Ethoxy-2-hydroxyphenyl)-1,3,5,6-tetraazainden
2-(2-Dimethylamino-4-methoxyphenyl)-1,3,5,6-tetraazainden
2-(4-Dimethylamino-2-methoxyphenyl)-1,3,5,6-tetraazainden, Fumarat, F. 226°
2-(2-Methoxy-4-trifluormethylphenyl)-1,3,5,6-tetraazainden
2-(4-Methoxy-2-trifluormethylphenyl)-1,3,5,6-tetraazainden
2-(2,3-Dimethoxyphenyl)-1,3,5,6-tetraazainden
2-(2,4-Dimethoxyphenyl)-1,3,5,6-tetraazainden, F. 268°
2-(2,5-Dimethoxyphenyl)-1,3,5,6-tetraazainden, Hydrochlorid, F. 290°
2-(2,6-Dimethoxyphenyl)-1,3,5,6-tetraazainden
2-(3,4-Dimethoxyphenyl)-1,3,5,6-tetraazainden, Hydrochlorid, F. 207°
2-(3,5-Dimethoxyphenyl)-1,3,5,6-tetraazainden
2-(2-Ethoxy-4-methoxyphenyl)-1,3,5,6-tetraazainden
2-(4-Ethoxy-2-methoxyphenyl)-1,3,5,6-tetraazainden
2-(2,4-Diethoxyphenyl)-1,3,5,6-tetraazainden
2-(2-Isopropyloxy-4-methoxyphenyl)-1,3,5,6-tetraazainden
2-(4-Isopropyloxy-2-methoxyphenyl)-1,3,5,6-tetraazainden
2-(2,4-Diisopropyloxyphenyl)-1,3,5,6-tetraazainden
2-(2-Allyloxy-4-methoxyphenyl)-1,3,5,6-tetraazainden
2-(4-Allyloxy-2-methoxyphenyl)-1,3,5,6-tetraazainden, Hydrochlorid, F. 271°
2-(4-Allyloxy-3-methoxyphenyl)-1,3,5,6-tetraazainden, Hydrochlorid, F. 280°
2-(2,4-Diallyloxyphenyl)-1,3,5,6-tetraazainden
2-(2-Hydroxy-4-propargyloxyphenyl)-1,3,5,6-tetraazainden
2-(4-Hydroxy-2-propargyloxyphenyl)-1,3,5,6-tetraazainden
2-(2-Methoxy-3-propargyloxyphenyl)-1,3,5,6-tetraazainden

2-(2-Methoxy-4-propargyloxyphenyl)-1,3,5,6-tetraazainden, F. 224°
2-(2-Methoxy-5-propargyloxyphenyl)-1,3,5,6-tetraazainden
2-(2-Methoxy-6-propargyloxyphenyl)-1,3,5,6-tetraazainden
2-(3-Methoxy-4-propargyloxyphenyl)-1,3,5,6-tetraazainden, Hydrochlorid, F. 259°
2-(4-Methoxy-2-propargyloxyphenyl)-1,3,5,6-tetraazainden
2-(2-Ethoxy-4-propargyloxyphenyl)-1,3,5,6-tetraazainden
2-(4-Ethoxy-2-propargyloxyphenyl)-1,3,5,6-tetraazainden
2-(2-Allyloxy-4-propargyloxyphenyl)-1,3,5,6-tetraazainden
2-(4-Allyloxy-2-propargyloxyphenyl)-1,3,5,6-tetraazainden
2-(2,4-Dipropargyloxyphenyl)-1,3,5,6-tetraazainden, Hydrochlorid, F. 265°
2-(2-Cyanmethoxy-4-hydroxyphenyl)-1,3,5,6-tetraazainden
2-(4-Cyanmethoxy-2-hydroxyphenyl)-1,3,5,6-tetraazainden
2-(2-Cyanmethoxy-4-methoxyphenyl)-1,3,5,6-tetraazainden
2-(4-Cyanmethoxy-2-methoxyphenyl)-1,3,5,6-tetraazainden, Hydrochlorid, F. 268°
2-(2-Cyanmethoxy-4-ethoxyphenyl)-1,3,5,6-tetraazainden
2-(4-Cyanmethoxy-2-ethoxyphenyl)-1,3,5,6-tetraazainden
2-(2-Allyloxy-4-cyanmethoxyphenyl)-1,3,5,6-tetraazainden
2-(4-Allyloxy-2-cyanmethoxyphenyl)-1,3,5,6-tetraazainden
2-(2-Cyanmethoxy-4-propargyloxyphenyl)-1,3,5,6-tetraazainden
2-(4-Cyanmethoxy-2-propargyloxyphenyl)-1,3,5,6-tetraazainden
2-(2,4-Bis-cyanmethoxy-phenyl)-1,3,5,6-tetraazainden
2-(2-Mercapto-4-methoxyphenyl)-1,3,5,6-tetraazainden
2-(4-Mercapto-2-methoxyphenyl)-1,3,5,6-tetraazainden
2-(2-Hydroxy-4-methylthiophenyl)-1,3,5,6-tetraazainden
2-(4-Hydroxy-2-methylthiophenyl)-1,3,5,6-tetraazainden
2-(2-Methoxy-4-methylthiophenyl)-1,3,5,6-tetraazainden, Hydrochlorid, F. 271°
2-(2-Methoxy-5-methylthiophenyl)-1,3,5,6-tetraazainden
2-(4-Methoxy-2-methylthiophenyl)-1,3,5,6-tetraazainden
2-(2-Ethoxy-4-methylthiophenyl),1,3,5,6-tetraazainden
2-(4-Ethoxy-2-methylthiophenyl)-1,3,5,6-tetraazainden
2-(2-Allyloxy-4-methylthiophenyl)-1,3,5,6-tetraazainden
2-(4-Allyloxy-2-methylthiophenyl)-1,3,5,6-tetraazainden
2-(2-Methylthio-4-propargyloxyphenyl)-1,3,5,6-tetraazainden
2-(4-Methylthio-2-propargyloxyphenyl)-1,3,5,6-tetraazainden
2-(2-Cyanmethoxy-4-methylthiophenyl)-1,3,5,6-tetraazainden
2-(4-Cyanmethoxy-2-methylthiophenyl)-1,3,5,6-tetraazainden
2-(2,4-Bis-methylthio-phenyl)-1,3,5,6-tetraazainden
2-(4-Ethylthio-2-methoxyphenyl)-1,3,5,6-tetraazainden, F. 248°
2-(4-Allylthio-2-methoxyphenyl)-1,3,5,6-tetraazainden
2-(2-Methoxy-4-propargylthiophenyl)-1,3,5,6-tetraazainden, F. 222°
2-(4-Cyanmethylthio-2-methoxyphenyl)-1,3,5,6-tetraazainden, F. 240°
2-(2-Hydroxy-4-methylsulfinylphenyl)-1,3,5,6-tetraazainden
2-(4-Hydroxy-2-methylsulfinylphenyl)-1,3,5,6-tetraazainden
2-(2-Methoxy-4-methylsulfinylphenyl)-1,3,5,6-tetraazainden
2-(4-Methoxy-2-methylsulfinylphenyl)-1,3,5,6-tetraazainden
2-(2-Ethoxy-4-methylsulfinylphenyl)-1,3,5,6-tetraazainden
2-(2-Ethoxy-2-methylsulfinylphenyl)-1,3,5,6-tetraazainden
2-(2-Allyloxy-4-methylsulfinylphenyl)-1,3,5,6-tetraazainden
2-(4-Allyloxy-2-methylsulfinylphenyl)-1,3,5,6-tetraazainden
2-(2-Methylsulfinyl-4-propargyloxyphenyl)-1,3,5,6-tetraazainden
2-(4-Methylsulfinyl-2-propargyloxyphenyl)-1,3,5,6-tetraazainden
2-(2-Cyanmethoxy-4-methylsulfinylphenyl)-1,3,5,6-tetraazainden
2-(4-Cyanmethoxy-2-methylsulfinylphenyl)-1,3,5,6-tetraazainden
2-(2,4-Bis-methylsulfinyl-phenyl)-1,3,5,6-tetraazainden
2-(4-Ethylsulfinyl-2-methoxyphenyl)-1,3,5,6-tetraazainden
2-(4-Allylsulfinyl-2-methoxyphenyl)-1,3,5,6-tetraazainden
2-(2-Methoxy-4-propargylsulfinyl)-1,3,5,6-tetraazainden
2-(4-Cyanmethylsulfinyl-2-methoxy)-1,3,5,6-tetraazainden
2-(2,3,4-Trimethoxyphenyl)-1,3,5,6-tetraazainden
2-(3,4,5-Trimethoxyphenyl)-1,3,5,6-tetraazainden
2-(3,5-Dimethoxy-4-propargyloxyphenyl)-1,3,5,6-tetraazainden.

Beispiel 2

Eine Lösung von 176 mg p-Propargyloxybenzoesäure in 1 ml Pyridin wird mit einer Lösung von 109 mg IIa in 1 ml Pyridin versetzt, wobei das entsprechende Salz ausfällt. Unter Rühren tropft man bei 0° 0,19 ml $SOCl_2$ hinzu, rührt 1 Stunde bei

0° und 1 Stunde bei 70°, dampft ein und nimmt mit verdünnter Salzsäure auf. Das erhaltene Hydrochlorid wird in Natriumcarbonatlösung eingetragen, das ausgefallene 2-p-Propargyloxyphenyl-1,3,4,7-tetraazainden wird abfiltriert. Fumarat, F. 273°.

Beispiel 3

Man kocht 2,06 g 2-Methoxy-4-propargyloxybenzoesäure mit 7 ml Benzol und 4 ml $SOCl_2$ 1 Stunde, dampft ein und löst in 5 ml Benzol. Die Lösung des Säurechlorids wird zu einem Gemisch aus 1,1 g IIb, 7 ml Pyridin und 5 ml Triethylamin getropft. Man rührt noch 2 Stunden bei 20°, versetzt mit Wasser, neutralisiert mit Salzsäure und arbeitet wie üblich auf. Das erhaltene

3-Amino-4-(2-methoxy-4-propargyloxybenzoylamino)-pyridazin

wird in das entsprechende Hydrochlorid übergeführt und dieses (200 mg) in 2 ml Pyridin gelöst. Unter Rühren bei 80° tropft man 0,2 ml $POCl_3$ hinzu, giesst nach 2 Stunden in Wasser, arbeitet wie üblich auf und erhält

2-(2-Methoxy-4-propargyloxyphenyl)-1,3,5,6-tetraazainden, F. 224°.

Beispiel 4

Ein Gemisch aus 11 g IIb, 19,6 g 2,4-Dimethoxybenzoesäuremethylester und 300 ml $POCl_3$ wird 2 Stunden auf 120° erhitzt, eingedampft und der Rückstand mit 2n Salzsäure behandelt. Man erhält 2-(2,4-Dimethoxyphenyl)-1,3,5,6-tetraazainden, F. 268°.

Beispiel 5

Ein Gemisch aus 1,1 g IIa, 1,63 g 2,4-Dimethoxybenzonitril und 3 g p-Toluolsulfonsäure-monohydrat wird 3,5-Stunden auf 160° erhitzt. Nach dem Abkühlen arbeitet man wie üblich auf und erhält 2-(2,4-Dimethoxyphenyl)-1,3,4,7-tetraazainden, F. 210–215°.

Beispiel 6

Man erhitzt ein Gemisch aus 4,33 g S-Methyl-2-methoxy-4-propargyloxy-thiobenzoesäure-morpholid-jodid (erhältlich durch Kochen von 2-Methoxy-4-propargyloxybenzaldehyd mit Schwefel in Morpholin und nachfolgende Umsetzung mit $CH_3J$ in Aceton), 1,1 g IIb und 35 ml Ethylenglykol 40 Minuten auf 130°, giesst in Eiswasser, filtriert und erhält

2-(2-Methoxy-4-propargyloxyphenyl)-1,3,5,6-tetraazainden, F. 224°.

Beispiel 7

Man erhitzt 1,1 g IIb und 3 g 2,4-Dimethoxybenzoesäureanhydrid 5 Stunden auf 180°, kühlt ab, arbeitet wie üblich auf und erhält 2-(2,4-Dimethoxyphenyl)-1,3,5,6-tetraazainden, F. 268°.

Beispiel 8

Man mischt 2,78 g 2-Methoxy-4-cyanmethoxybenzoesäuremorpholid mit 1,1 g IIb, tropft unter Rühren 5 ml $POCl_3$ hinzu, kocht 3 Stunden und dampft ein. Nach üblicher Aufarbeitung erhält man

2-(4-Cyanmethoxy-2-methoxyphenyl)-1,3,5,6-tetraazainden, Hydrochlorid, F. 268°.

Beispiel 9

Man erhitzt 1,1 g IIa, 2,87 g 2,4-Dimethoxybenzoesäure-morpholid-imidchlorid, 6 ml Triethylamin und 5 ml Diethylenglykol-dimethylether 30 Minuten auf 120°. Nach dem Erkalten arbeitet man wie üblich auf und erhält

2-(2,4-Dimethoxyphenyl)-1,3,4,7-tetraazainden, F. 210–215°.

Beispiel 10

Man rührt 11 g IIa, 19 g 2-Methoxy-4-propargyloxybenzaldehyd (erhältlich aus 2,4-Dihydroxybenzaldehyd über 2-Hydroxy-4-propargyloxybenzaldehyd) und 10 g Schwefel in 200 ml Mesitylen 10 Stunden bei 180°, dampft ein, extrahiert mit Methanol, filtriert und konzentriert die Lösung auf 350 ml. Durch Zugabe von etherischer Salzsäure fällt

2-(2-Methoxy-4-propargyloxyphenyl)-1,3,4,7-tetraazainden-hydrochlorid

aus; freie Base, F. 189–190°.

Beispiel 11

Man löst 11 g IIb und 18,9 g 2-Methoxy-4-cyanmethoxybenzaldehyd in 100 ml Dimethylacetamid, rührt nach Zugabe von 19 g Natriumdisulfit zwei Stunden bei 140°, arbeitet wie üblich auf und erhält

2-(4-Cyanmethoxy-2-methoxyphenyl)-1,3,5,6-tetraazainden, Hydrochlorid, F. 268°.

Analog Beispiel 10 oder 11 erhält man aus IIa oder IIb mit den entsprechenden Aldehyden (wie z.B. p-Propargyloxybenzaldehyd, F. 80°) die übrigen in Beispiel 1 angegebenen Verbindungen.

Beispiel 12

Man löst 24,1 g 2-(4-Hydroxy-2-methoxyphenyl)-1,3,5,6-tetraazainden in der berechneten Menge 2n Natronlauge, dampft ein und entfernt Reste von Wasser durch zweimalige Zugabe von Toluol und Eindampfen. Das erhaltene Na-Salz wird in 300 ml Dimethylformamid aufgenommen, mit 8 ml Propargylchlorid versetzt und 16 Stunden bei 20° gerührt. Man arbeitet mit Wasser und Ethylacetat wie üblich auf und erhält 2-(2-Methoxy-4-propargyloxyphenyl)-1,3,5,6-tetraazainden, F. 224°.

Analog erhält man aus den entsprechenden Hydroxyverbindungen durch Veretherung die übrigen in Beispiel 1 angegebenen Ether.

Beispiel 13

Ein Gemisch von 10 g 2-(4-Acetoxy-2-methoxyphenyl)-1,3,4,7-tetraazainden (erhältlich durch Kondensation von IIa mit 4-Acetoxy-2-methoxybenzoesäure), 100 ml Methanol und 100 ml 2n wässeriger NaOH-Lösung wird 16 Stunden bei 20° stehengelassen. Nach üblicher Aufarbeitung erhält man 2-(4-Hydroxy-2-methoxyphenyl)-1,3,4,7-tetraazainden.

Analog erhält man durch alkalische Hydrolyse der entsprechenden Acetoxyverbindungen die übrigen in Beispiel 1 genannten Hydroxyverbindungen.

Beispiel 14

Man hydriert eine Lösung von 10 g 2-(4-Benzyloxy-2-methoxyphenyl)-1,3,5,6-tetraazainden-hydrochlorid (erhältlich durch Kondensation von IIb mit 4-Benzyloxy-2-methoxy-benzoesäure) in 150 ml Methanol an 5 g 5%igem Pd-C bei 20° und 1 bar bis zum Ende der Wasserstoffaufnahme, filtriert, arbeitet wie üblich auf und erhält 2-(4-Hydroxy-2-methoxyphenyl)-1,3,5,6-tetraazainden.

Analog erhält man durch Hydrogenolyse der entsprechenden Benzylehter bzw. Benzylthioether die übrigen in Beispiel 1 genannten Hydroxy- bzw. Mercaptoverbindungen.

Beispiel 15

Analog Beispiel 12 erhält man durch Umwandlung von 2-(4-Mercapto-2-methoxyphenyl)-1,3,5,6-tetraazainden in das Na-Salz und nachfolgende Reaktion mit Ethyljodid das 2-(2-Methoxy-4-ethylthiophenyl)-1,3,5,6-tetraazainden; F. 248°.

Analog erhält man aus den entsprechenden Mercaptoverbindungen durch Thio-veretherung die übrigen in Beispiel 1 beschriebenen Thioether.

Beispiel 16

Zu einer siedenden Lösung von 2,84 g 2-(4-Ethylthio-2-methoxyphenyl)-1,3,5,6-tetraazainden in 50 ml Ethanol gibt man 10 ml 30%iges $H_2O_2$ und kocht anschliessend 3 Stunden. Nach dem Abkühlen und üblicher Aufarbeitung erhält man 2-(4-Ethylsulfinyl-2-methoxyphenyl)-1,3,5,6-tetraazainden.

Analog erhält man durch Oxydation der entsprechenden Thioether die übrigen in Beispiel 1 beschriebenen Sulfinylverbindungen.

Die nachstehenden Beispiele betreffen pharmazeutische Zubereitungen, die Verbindungen der Formel I oder ihre Säureadditionssazle enthalten:

Beispiel A: Tabletten

Ein Gemisch von 1 kg 2-(2,4-Dimethoxyphenyl)-1,3,5,6-tetraazainden, 4 kg Lactose, 1,2 kg Kartoffelstärke, 0,2 kg Talk und 0,1 kg Magnesiumstearat wird in üblicher Weise zu Tabletten gepresst, derart, dass jede Tablette 100 mg Wirkstoff enthält.

Beispiel B: Dragees

Analog Beispiel A werden Tabletten gepresst, die anschliessend in üblicher Weise mit einem Überzug aus Saccharose, Kartoffelstärke, Talk, Tragant und Farbstoff überzogen werden.

Beispiel C: Kapseln

10 kg 2-(2-Methoxy-4-propargyloxyphenyl)-1,3,5,6-tetraazainden werden in üblicher Weise in Hartgelatinekapseln gefüllt, so dass jede Kapsel 50 mg Wirkstoff enthält.

Beispiel D: Ampullen

Eine Lösung von 1 kg 2-(2,4-Dimethoxyphenyl)-1,3,4,7-tetraazainden-fumarat in 100 l zweifach destilliertem Wasser wird steril filtriert, in Ampullen abgefüllt, unter sterilen Bedingungen lyophilisiert und steril verschlossen. Jede Ampulle enthält 50 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

Bei der Untersuchung der positiv inotropen Wirkung am isolierten Vorhof des Meerschweinchens (Methodik in Anlehnung an Arzneimittelforschung, 1.c., Seite 162) ergaben sich für einige charakteristische Verbindungen der Formel I folgende relative Wirkungsstärken im Vergleich zu Amrinon:

| Substanz | | Wirkungs-stärke |
|---|---|---|
| Amrinon (Vergleichssubstanz) | | |
| Formel I, | | |
| A= | Ar= | |
| –CH=N–N=CH– | 2-Methoxy-4-propargyloxyphenyl | 70 |
| –N=CH–CH=N– | 2,4-Dimethoxyphenyl | 41 |
| –N=CH–CH=N– | 2-Methoxy-4-propargyloxyphenyl | 41 |
| –CH=N–N=CH– | 4-Cyanmethoxy-2-methoxyphenyl | 22 |
| –CH=N–N=CH– | 2,4-Dimethoxyphenyl | 20 |
| –N=CH–CH=N– | p-Propargyloxyphenyl | 12 |

**Patentansprüche**

1. 2-Aryl-tetraazaindene der allgemeinen Formel I

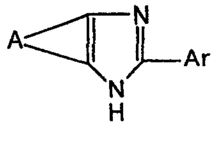

I

worin

–A– –N=CH–CH=N– oder –CH=N–N=CH–,

Ar einen ein- bis dreifach durch Hydroxy-, Mercapto-, Dialkylamino-, Trifluormethyl- und/oder –Z–R-Gruppen substituierten Phenylrest,

Z –O–, –S– oder –SO– und

R Alkyl, Alkenyl, Alkinyl oder Cyanmethyl bedeuten,
wobei die Alkyl-, Alkenyl- und Alkinylgruppen jeweils bis zu 5 C-Atome besitzen
sowie ihre physiologisch unbedenklichen Salze.

2.

a) 2-(2,4-Dimethoxyphenyl)-1,3,4,7-tetraazainden.
b) 2-(2-Methoxy-4-propargyloxyphenyl)-1,3,4,7-tetraazainden.
c) 2-(4-Cyanmethoxy-2-methoxyphenyl)-1,3,4,7-tetraazainden.
d) 2-(2,4-Dimethoxyphenyl-1,3,5,6-tetraazainden.
e) 2-(2-Methoxy-4-propargyloxyphenyl)-1,3,5,6-tetraazainden.
f) 2-(4-Cyanmethoxy-2-methoxyphenyl)-1,3,5,6-tetraazainden.

3. Verfahren zur Herstellung von 2-Aryltetraazaindenen der allgemeinen Formel I

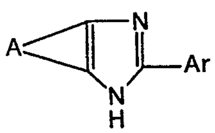
I

worin
–A– –N=CH–CH=N– oder –CH=N–N=CH–,
Ar einen ein- bis dreifach durch Hydroxy-, Mercapto-, Dialkylamino-, Trifluormethyl- und/oder –Z–R-Gruppen substituierten Phenylrest,
Z –O–, –S– oder –SO– und
R Alkyl, Alkenyl, Alkinyl oder Cyanmethyl bedeuten,
wobei die Alkyl-, Alkenyl- und Alkinylgruppen jeweils bis zu 5 C-Atome besitzen,
sowie ihrer physiologisch unbedenklichen Salze, dadurch gekennzeichnet, dass man ein Diamin der allgemeinen Formel II

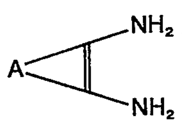
II

worin die Gruppe
–A– die oben angegebene Bedeutung hat mit einer Benzoesäure der allgemeinen Formel III

HOOC–Ar III

worin
Ar die oben angegebenen Bedeutung hat
oder mit einem ihrer reaktionsfähigen Derivate oder mit einem Aldehyd der allgemeinen Formel IV

OCH–Ar IV

worin
Ar die oben angegebene Bedeutung hat
in Gegenwart eines Oxydationsmittels
umsetzt
oder dass man eine Verbindung, die der Formel I entspricht, aber anstelle einer oder mehrerer freier Hydroxy-, und/oder Mercaptogruppen eine oder mehrere geschützte Hydroxy- und/oder Mercaptogruppen enthält, mit einem solvolysierenden oder hydrogenolysierenden Mittel behandelt
und dass man gegebenenfalls in dem erhaltenen Produkt Hydroxygruppen verethert und/oder Mercaptogruppen in Thioethergruppen überführt und/oder Thioethergruppen zu Sulfinylgruppen oxydiert und/oder eine erhaltene Verbindung durch Behandeln mit einer Säure in eines ihrer physiologisch unbedenklichen Salze umwandelt.

4. Verfahren zur Herstellung pharmazeutischer Zubereitungen, dadurch gekennzeichnet, dass man eine Verbindung der Formel I gemäss Anspruch 1 und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I gemäss Anspruch 1 und/oder einem ihrer physiologisch unbedenklichen Salze.

6. Verbindungen der allgemeinen Formel I gemäss Anspruch 1 zur Bekämpfung von Krankheiten.

## Claims

1. 2-Aryltetraazaindenes of the general formula I

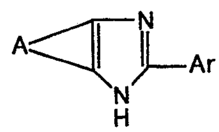
I

in which
–A– is –N=CH–CH=N– or –CH=N–N=CH–,
Ar is a phenyl radical which is mono- to trisubstituted by hydroxyl, mercapto, dialkylamino. trifluoromethyl and/or –Z–R groups,
Z is –O–, –S– or –SO– and
R is alkyl, alkenyl, alkynyl or cyanomethyl, the alkyl, alkenyl and alkynyl groups each having up to 5 C atoms, and their physiologically acceptable salts.

2.

a) 2-(2,4-Dimethoxyphenyl)-1,3,4,7-tetraazaindene.
b) 2-(2-Methoxy-4-propargyloxyphenyl)-1,3,4,7-tetraazaindene.
c) 2-(4-Cyanomethoxy-2-methoxyphenyl)-1,3,4,7-tetraazaindene.
d) 2-(2,4-Dimethoxyphenyl)-1,3,5,6-tetraazaindene.
e) 2-(2-Methoxy-4-propargyloxyphenyl)-1,3,5,6-tetraazaindene.
f) 2-(4-Cyanomethoxy-2-methoxyphenyl)-1,3,5,6-tetraazaindene.

3. Process for preparing 2-aryltetraazaindenes of the general formula I

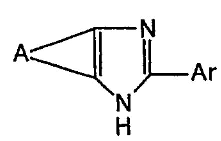
I

in which
–A– is –N=CH–CH=N– or –CH=N–N=CH–,
Ar is a phenyl radical which is mono- to trisubstituted by hydroxyl, mercapto, dialkylamino, trifluoromethyl and/or –Z–R groups,

Z is –O–, –S– or –SO– and

R is alkyl, alkenyl, alkynyl or cyanomethyl, the alkyl, alkenyl and alkynyl groups each having up to 5 C atoms, and of their physiologically acceptable salts, characterised in that a diamine of the general formula II

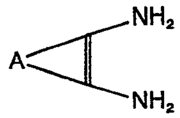
II

in which the group –A– is as defined above, is reacted with a benzoic acid of the general formula III

HOOC–Ar III

in which Ar is as defined above, or with one of its reactive derivatives or with an aldehyde of the general formula IV

OCH–Ar IV

in which Ar is as defined above, in the presence of an oxidising agent, or that a compound which has the formula I but instead of one or more free hydroxyl and/or mercapto groups contains one or more protected hydroxyl and/or mercapto groups is treated with a solvolysing or hydrogenolysing agent, and that, if appropriate, the product obtained has its hydroxyl groups etherified and/or its mercapto groups converted into thioether groups and/or its thioether groups oxidised to sulfinyl groups and/or a compound obtained is converted into one of its physiologically acceptable salts by treating it with an acid.

4. Process for preparing pharmaceutical preparations, characterised in that a compound of the formula I according to claim 1 and/or one of its physiologically acceptable salts are brought into a suitable form of administration together with at least one solid, liquid or semi-liquid carrier or adjunct and, if appropriate, in a combination with one or more other active ingredients.

5. Pharmaceutical preparation, characterised by containing at least one compound of the formula I, according to claim 1 and/or one of its physiologically acceptable salts.

6. Compounds of the general formula I, according to claim 1 for treating diseases.

**Revendications**

1. 2-aryl-tétra-aza-indènes de la formule générale I

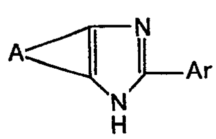
I

où

–A– signifie –N=CH–CH=N ou –CH=N–N=CH–,

Ar signifie un radical phénylique substitué 1 à 3 fois par des groupes hydroxy, mercapto, dialkylamino, trifluorométhyle et/ou –Z–R,

Z représente –O–, –S ou –SO et

R représente un alkyle, alkényle, alkinyle ou cyanométhyle

où les groupes alkyle, alkényle et alkinyle possèdent chacun 5 atomes de carbone au maximum ainsi que leurs sels sans nocivité physiologique.

2.

a) le 2-(2,4-diméthoxyphényl)-1,3,4,7-tétra-aza-indène.
b) le 2-(2-méthoxy-4-propargyloxyphényl)-1,3,4,7-tétra-aza-indène.
c) le 2-(4-cyanométhoxy-2-méthoxyphényl)-1,3,4,7-tétra-aza-indène.
d) le 2-(2,4-diméthoxyphényl)-1,3,5,6-tétra-aza-indène.
e) le 2-(2-méthoxy-4-propargyloxyphényl)-1,3,5,6-tétra-aza-indène.
f) le 2-(4-cyanométhoxy-2-méthoxyphényl)-1,3,5,6-tétra-aza-indène.

3. Procédé pour la préparation de 2-aryl-tétra-aza-indènes de la formule générale I

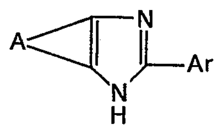
I

où

–A– représente –N=CH–CH=N– ou –CH=N–N=CH–,

Ar représente un radical phénylique substitué une à trois fois par des groupes hydroxy, mercapto, dialkyle, amino, trifluorométhyle et/ou –Z–R–,

Z représente –O–, –S– ou –SO– et

R représente un alkyle, un alkényle, un alkinyle ou un cyanométhyle

où les groupes alkyle, alkényle et alkinyle possèdent chacun 5 atomes de carbone au maximum ainsi que leurs sels sans nocivité physiologique, caractérisé en ce que l'on transforme une diamine de la formule générale II

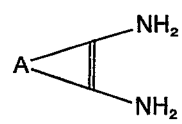
II

où le groupe

–A– a la même signification que ci-dessus avec un acide benzoïque de la formule générale III

HOOC–Ar III

où

Ar a la même signification que ci-dessus

ou avec un de ses dérivés susceptibles de réaction ou avec un aldéhyde de la formule générale IV

OCH–Ar IV

où

Ar a la même signification que ci-dessus

en présence d'un agent oxydant

ou en ce que l'on traite un composé correspondant à la formule I mais qui possède, à la place d'un ou plusieurs groupes libres hydroxy ou mercapto, un ou plusieurs groupes protégés hydroxy et/ou mercapto avec un agent solvolisant ou hydrogénolysant

et en ce que l'on soumet éventuellement les groupes hydroxy du produit obtenu à éthérification et/ou que l'on transforme les groupes mercapto en groupes thioéther et/ou que l'on oxyde les groupes thioéther en groupes sulfinyles et/ou que l'on transforme un ccmposé obtenu en un de ses sels sans nocivité physiologique par traitement avec un acide.

4. Procédé pour la production de préparations pharmaceutiques caractérisé en ce que l'on met, sous une forme de dosage appropriée, un composé de la formule I, selon la revendication 1, et/ou un de ses sels sans nocivité physiologique, avec addition d'au moins un excipient ou un adjuvant solide, liquide ou semi-liquide et, éventuellement en combinaison avec une ou plusieurs autres substances actives.

5. Préparation pharmaceutique caractérisée par une teneur en au moins un composé de la formule I selon la revendication 1 et/ou un des ses sels sans nocivité physiologique.

6. Composé de la formule générale I selon la revendication 1, pour lutter contre les maladies.